# EUROPÄISCHE PATENTSCHRIFT

(11) **EP 0 508 219 B1**
(45) Veröffentlichungstag und Bekanntmachung des Hinweises auf die Patenterteilung: **26.04.2000**
(21) Anmeldenummer: 92105128.0
(22) Anmeldetag: 17.12.1984
(51) Int. Cl.: C07D 498/10, G03C 1/72

(54) **Photochrome Substanzen**
Photochromic substances
Substances photochromiques

(30) Priorität: 16.12.1983 DE 3345625; 16.12.1983 DE 3345624
(43) Veröffentlichungstag der Anmeldung: 14.10.1992
(62) Teilanmeldung aus: 84115602.9
(73) Patentinhaber: Optische Werke G. Rodenstock, 80469 München (DE)
(72) Erfinder: Melzig,Manfred,Dr., W-8031 Wessling (DE); Martinuzzi,Giuseppe, W-8031 Eichenau (DE)
(74) Vertreter: Münich, Wilhelm, Dr.

(56) Entgegenhaltungen:
- GB-A- 2 029 410
- US-A- 3 578 602

## Beschreibung

Die Erfindung bezieht sich auf photochrome Substanzen, d.h. auf Substanzen, deren Einfärbung sich entsprechend der Umgebungsbeleuchtung in Intensität und Farbeindruck ändert.

Photochrome Substanzen haben eine Vielzahl von Anwendungsmöglichkeiten, beispielsweise werden sie zur Herstellung von photochromen Sonnenschutz-Brillengläsern aus Kunststoff, Brillenfassungen etc. verwendet.

Photochrome Substanzen sind beispielsweise aus den US-PSen US-A-3 562 172, US-A-3 578 602, US-A-4 215 010 oder der DE-A-1 927 849, der DE-A-29 26 255 oder der GB-A-2 029 410 bekannt.

Die aus diesen Druckschriften bekannten photochromen Substanzen bzw. Verbindungen haben jedoch unter anderem den Nachteil, daß der photochrome Effekt stark temperaturabhängig ist: Mit steigenden Umgebungstemperaturen wird die Eindunkelung bei gleicher Beleuchtungsstärke geringer.

Der Erfindung liegt die Aufgäbe zugrunde, photochrome Verbindungen anzugeben, deren photochromer Effekt sich auch mit steigender Temperatur nur wenig ändert, d.h. die auch bei steigender Temperatur gute Eindunkelungseigenschaften aufweisen.

Diese Aufgabe wird erfindungsgemäß durch Verbindungen mit der allgemeinen Formel 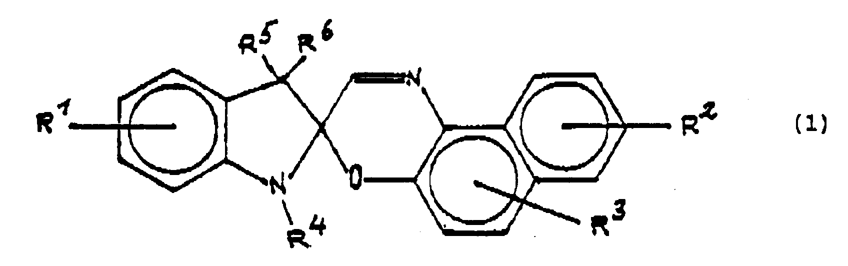 worin
- R¹,R²,R³: einen oder mehrere Substituenten aus der Reihe -H, -CN, -NO₂, -X, -CH₂X, -CH₂X, -CX₃, -OR, -COR, -COOR bedeuten,
wobei X = Halogen und R = Alkyl mit bis zu 8 C-Atomen, Aryl bzw. annelierte aromatische Ringsysteme sind,
- R⁴,R⁵,R⁶: ein Substituent aus der Reihe -R, -CH₂R, ist,
wobei R = Alkyl mit bis zu 8 C-Atomen oder Aryl bedeutet, wobei die Reste R⁵ und R⁶ nicht beide Methyl sind.

Erfindungsgemäß ist erkannt worden, daß für die Verringerung der Temperaturabhängigkeit des photochromen Effekts die Reste R⁴, R⁵ und R⁶ entscheidend sind:

Durch die Einwirkung von kurzwelligem Licht öffnet sich die Spiro-C-O Bindung; nach der von den Erfindern entwickelten Modellvorstellung entstehen aus der Grundform, d.h. dem Molekül mit der allgemeinen Formel (1) folgende Moleküle, die miteinander im Gleichgewicht stehen:

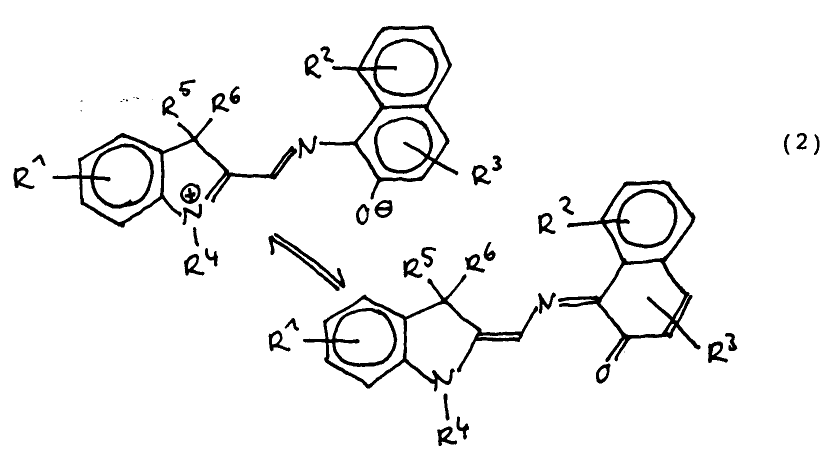

Die Moleküle mit der Strukturformel (2) haben eine blaue Farbe, während das Molekül mit der Strukturformel (1) farblos ist.

Durch steigende Temperaturen wird die Rückreaktion begünstigt, d.h. das Gleichgewicht zwischen Molekülen mit der Strukturformel (1) und (2) wird bei gleicher Beleuchtungsstärke durch steigende Temperatur von der Modifikation (2) zu der Grundform (1) hin verschoben.

Sind die Reste R⁵ und R⁶ "größer" als Methyl, so weisen die erfindungsgemäßen photochromen Substanzen den zusätzlichen Vorteil der positiven Thermochromie auf, d.h. die Eindunkelung nimmt mit steigender Temperatur zu. Nach einer ebenfalls von den Erfindern entwickelten Modellvorstellung öffnet sich bei Verbindungen mit positiver Thermochromie ebenfalls die Spiro-C-O-Bindung, und es dürften folgende Moleküle entstehen, die eine blau-violette Farbe haben:

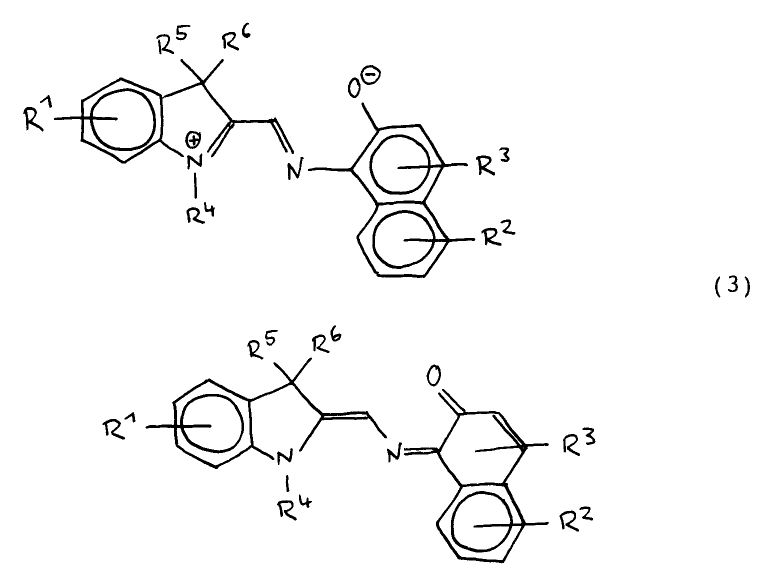

Das Gleichgewicht zwischen der Grundform (1) und der Modifikation (3) hängt nur von der Temperatur, nicht jedoch von der Beleuchtungsstärke ab.

Solche photo- und thermochromen Verbindungen sind in einer Matrix, beispielsweise einem optischen Element, ausschließlich phototropen Verbindungen, wie sie beispielsweise in der US-A-3 578 602 beschrieben sind, auch unter der Annahme gleicher phototroper Eigenschaften dadurch überlegen, daß sie im Sonnenlicht durch das durch dieses bewirkte Aufheizen der Matrix eine stärkere Eindunkelung aufweisen.

Die erfindungsgemäßen photochromen Substanzen haben den zusätzlichen Vorteil, daß bei gleicher Konzentration der Moleküle in einer Matrix, beispielsweise einem optischen Element, die Eindunkelung größer ist als bei den Substanzen, die in den eingangs genannten Durckschriften beschrieben sind.

Die Synthese der erfindungsgemäßen photochromen Verbindung erfolgt in an sich bekannter Weise durch Kondensation eines substituierten 2-Alkylenindolins 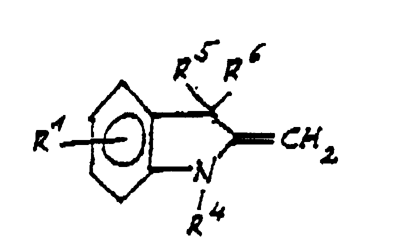 mit einen 2-Hydroxy-1-nitroso-aromaten

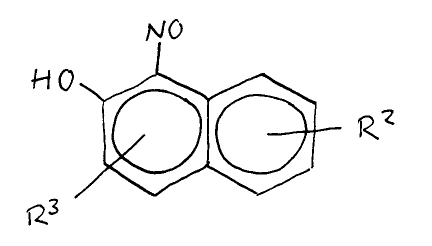

Die Bildung des 2-Alkylenindolins kann durch vorhergehende Synthese oder während des letzten Syntheseschrittes aus einem entsprechenden Indoleninium Salz erfolgen.

Eine vorteilhafte Weiterbildung ist im Anspruch 2 angegeben.

In den Ansprüchen 3 und 4 sind Möglichkeiten gekennzeichnet, wie optische Elemente mit der erfindungsgemäßen photochromen Verbindung hergestellt werden können.

In besonders vorteilhafter Weise lassen sich die erfindungsgemäßen photochromen Substanzen bei einem Sonnenschutz-Brillenglas aus einem Kunststoffmaterial einsetzen.

Im folgenden sollen zwei Beispiele für erfindungsgemäße photochrome Substanzen vorgestellt werden:

### Beispiel 1

20,3g (0,1mol) 7-Methoxy-1-nitroso-2-naphthol wird in 150 ml Trichlorethylen suspendiert, mit 4,25g (0,05 mol) Piperidin versetzt und unter Rückfluß bis zur vollständigen Lösung des Naphthols gekocht.

21,5 g (0,1 mol) 3,3-Diethyl-2-methylen-1-methylindolin werden in 100 ml Trichlorethylen gelöst und langsam zur ersteren Lösung bei Siedehitze zugetropft. Das Gemisch wird anschließend noch ca. 3 Stunden unter Rückfluß gekocht.

Die Lösung wird auf ca. 1/3 ihres Volumens eingeengt und über Al₂O₃ mit Trichlorethylen als Laufmittel chromatographiert. Aus dem ersten Eluat kann die nicht umgesetzte basische Ausgangsverbindung zurückgewonnen werden, die grüne Hauptzone enthält das Produkt. Das Laufmittel wird im Vakuum abgezogen. Aus den Rückstand kristallisiert nach längerem Stehenlassen rohes 3,3 Diethyl 1 methyl spiro (indolino 9'-methoxy-2,3' (3H)naphth(2.1-b)(1.4)oxazin), das durch mehrfaches Umkristallisieren auf Methanol gereinigt wird. Der Schmelzpunkt der erhaltenen Verbindung liegt bei 138°C, die Kristalle sind schwach gelblich. Die Verbindung hat dabei die folgenden 1H-NMR-Daten in ppm:

| | | | | |
|---|---|---|---|---|
| δ(-CH₂-CH₃-) | = 0,74 und 0,87 | (T) | R⁵, | R⁶ |
| δ(-CH₂-) | = 1,80 und 1,99 | (DQ) | R⁵, | R⁶ |
| δ(N-CH₃) | = 2,63 | (S) | R⁴ | |
| δ(O-CH₃) | = 4,00 | (S) | R² | |

Entsprechend diesen NMR-Daten hat die Verbindung die allgemeine Formel:

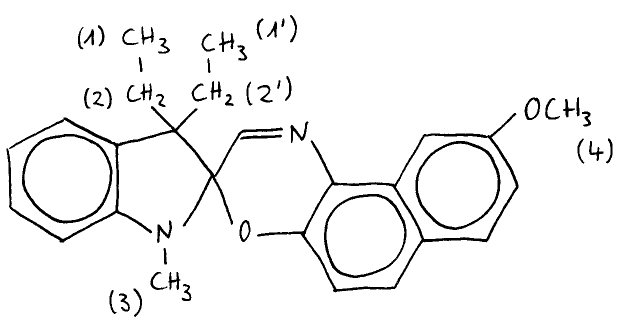

### Beispiel 2

20,3g (0,1 mol) 7-Methoxy-1-nitroso-2-naphtol wird in 150 ml Acetonitril suspendiert, mit 4,25 g (0,05 mol) Piperidin versetzt und unter Rückfluß bis zur vollständigen Lösung des Naphthols gekocht.

20,1g (0,1 mol) 1-Methyl-2-methylen-3-spirocyclo-hexylindolin werden in 150 ml Acetonitril gelöst und bei Siedehitze zur ersteren Lösung zugetropft.

Die weitere Behandlung erfolgt analog Beispiel 1, vor der chromatographischen Reinigung wird allerdings das Lösungsmittel vollständig abdestilliert.

Das gereinigte 1-Methyl-3-spirocyclohexyl-spiro (indolino-2,3'-(3H)naphth(2.1-b)(1.4)oxazin) besitzt einen Schmelzpunkt von 168°C und folgende 1H-NMR-Daten in ppm:

| | | | | |
|---|---|---|---|---|
| δ(-CH₂-) | = 1,60 | (M) | R⁵, | R⁶ |
| δ(N-CH₃) | = 2,68 | (S) | R⁴ | |
| δ(0-CH₃) | = 3,90 | (S) | R² | |

Entsprechend diesen NMR-Daten hat die Verbindung die Formel:

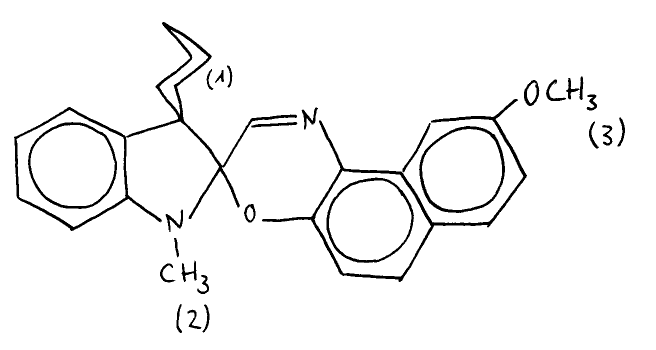

Im folgenden soll exemplarisch eine Möglichkeit beschrieben werden, wie erfindungsgemäße Verbindungen, beispielsweise die Substanzen, deren Herstellung vorstehend beschrieben worden ist, in eine Matrix eingebracht werden können.

Bei den folgenden Ausführungsbeispielen wird als Matrix eine 2mm dicke Planlinse aus Diethylenglykol-bis-allylcarbonat verwendet, das beispielsweise unter dem Warenzeichen CR 39 vertrieben wird.

Durch Einrühren von ca 5 Gew.% des Farbstoffs in ein Silikonöl oder in ein ähnliches hochsiedendes inertes Lösemittel wird ein Färbebad bereitet.

Die Kunststofflinse wird nach entsprechender Vorwärmung ca 20 min. lang in dieses Bad getaucht. Die Temperatur des Bads richtet sich nach dem Schmelzpunkt der photochromen Verbindung, sie liegt etwa 10°C über diesen. Da Temperaturen über 200°C die Matrix zerstören, werden höher schmelzende Substanzen bei 180°C max. 30 min eingebracht. Nach dem Abkühlen wird die Linse mit Methylenchlorid gewaschen, der Farbstoff ist beidseitig in das Kunststoffmaterial eindiffundiert. Diese Einfärbung kann gegebenenfalls mehrfach wiederholt werden.

Die mit den erfindungsgemäßen photochromen Verbindungen eingefärbten Kunststofflinsen zeigen folgendes Transmissionsverhalten unter Ausschluß kurzwelligen Lichts, d.h. ohne Beleuchtung, wobei die Transmission bei 10°C als Bezugswert gewählt wird.

**Tabelle 1**

| Anstieg der Lichtabsorption (%) | | | |
|---|---|---|---|
| Temperatur | Beispiel 1 | Beispiel 3 | Vergleichssubstanz 1 |
| 10°C | | | |
| 20°C | + 0,3 | + 0,2 | + 0,1 |
| 30°C | + 1,5 | + 0,7 | + 0,1 |
| 40°C | + 2,8 | + 2,9 | + 0,2 |
| 50°C | + 4,3 | + 5,1 | + 0,2 |
| 60°C | + 6,3 | + 6,9 | + 0,3 |

Die in Tabelle 1 aufgeführten Verbindungen haben folgende Reste in der allgemeinen Formel (1):

| | R¹,R³ | R² | R⁴ | R⁵ | R⁶ |
|---|---|---|---|---|---|
| Beispiel 1 | H | 9-0CH₃ | CH₃ | C₂H₅ | C₂H₅ |
| Beispiel 3 | H | 9-0CH₃ | C₂H₅ | C₂H₅ | C₂H₅ |
| Vergleichssub.1 | H | 9-0CH₃ | CH₃ | CH₃ | CH₃ |

Die zur Messung verwendete Vergleichssubstanz 1 ist in der GB-A-2 029 410 bzw. US-A-4 215 010 als erstes Beispiel in Tabelle II auf Seite 2 beschrieben. Diese Substanz entspricht der auf Seite 1 der GB-A-2 029 410 gezeigten allgemeinen Formelzeichnung, wobei die Reste R³, R⁴ = H und R¹ = Methoxy bedeuten.

Zur Messung der Temperaturabhängigkeit des photochromen Effekts wurden die Linsen nach dem oben beschriebenen Verfahren so eingefärbt, daß der nach V -bewertete Transmissionsunterschied zwischen unbelichtetem und belichtetem Glas, bei der Standardtemperatur vom 23°C (DIN 58217) gemessen, annähernd gleich war. Die Messung erfolgte in einer Kinetikbank mit temperierbarer Küvette, die Belichtung erfolgte unter einem Winkel von 30° mit einer Beleuchtungsstärke von 60 000 lux. Das Spektrum der verwendeten Xenonhochdrucklampe war durch Filterkombinationen dem Spektrum des natürlichen Sonnenlichts auf Meereshöhe weitgehend angepaßt. Gemessen wurde die nach der spektralen Empfindlichkeit des menschlichen Auges bewertete Transmission nach 15-minütiger Bestrahlung. Zum Ausbleichen wurden die Gläser 30 min bei 90°C ausgeheizt und anschließend im Dunkeln auf Raumtemperatur abgekühlt.

**Tabelle 2**

| Unterschied in der Lichtdurchlässigkeit | | | |
|---|---|---|---|
| Temp. | Beisp. 1 | Vergl. subst.1 | Vergl.-subst.2 |
| 10°C | 62,5 | 65,5 | 72,5 |
| 20°C | 52,5 | 52 | 61,5 |
| 30°C | 36,5 | 31 | 35,5 |
| Δ(Δτ) | 26 | 34 | 37 |

Die in Tabelle 2 aufgeführten Verbindungen haben folgende Reste in der allgemeinen Formel (1) (die Vergleichssustanzen sind der US-A-3 578 602 und der US-A-4 215 010 entnommen) :

| | | R^{1,3} | R² | R⁴ | R⁵ | R⁶ |
|---|---|---|---|---|---|---|
| Beispiel 1 | | H | 9-OCH₃ | CH₃ | C₂H₅ | C₂H₅ |
| Vergleichss. | 1 | H | H | CH₃ | CH₃ | CH₃ |
| | 2 | H | 9-OCH₃ | CH₃ | CH₃ | CH₃ |

Wie aus der Tabelle 2 ersichtlich ist, zeigen die erfindungsgemäßen Verbindungen bei gleicher oder sogar geringerer Eindunkelung als die bislang bekannten Verbindungen bei 10°C, einen stärkeren photochromen Effekt als diese bei 30°C. Der störende Abfall der Eindunkelung mit steigender Temperatur bei gleicher Beleuchtungsstärke, als Δ(Δτ) zwischen 10°C und 20°C und 30°C bewertet, wird um bis zu 40% reduziert.

## Patentansprüche

1. Verbindungen der allgemeinen Formel 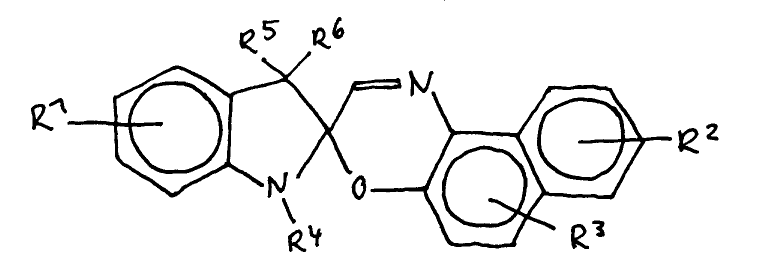 worin
R¹ ,R² ,R³ einen oder mehrere Substituenten aus der Reihe -H, -CN, -NO₂, -X, -CH₂X, -CH₂X, -CX₃, -OR, -COR, -COOR bedeuten,
wobei X = Halogen und R = Alkyl mit bis zu 8 C-Atomen sind,
R⁴ ,R⁵ ,R⁶ ein Substituent aus der Reihe -R, -CH₂R, ist
wobei R = Alkyl mit bis zu 8 C-Atomen oder Aryl bedeutet, wobei die Reste R⁵ und R⁶ nicht beide Methyl sind.

2. Verbindung mit folgender Formel:

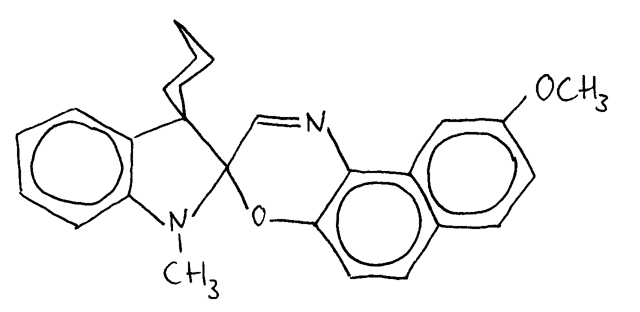

3. Optisches Element,
dadurch **gekennzeichnet,** daß es eine wirksame Menge einer photochromen Verbindung nach Anspruch 1 oder 2 enthält, die in das optische Element eingebracht oder als Schicht auf einem Kunststoffträgermaterial aufgebracht ist.

4. Optisches Element nach Anspruch 3,
dadurch **gekennzeichnet**, daß das optische Element aus einem Kunststoffmaterial besteht.

5. Optisches Element nach Anspruch 3 oder 4,
dadurch **gekennzeichnet,** daß das Element dadurch hergestellt ist,
daß die photochrome Verbindung in ein Silikonöl oder ein ähnliches hochsiedendes inertes Lösemittel eingerührt wird,
daß die Mischung auf eine Temperatur etwa 10° oberhalb des Schmelzpunktes der photochromen Verbindung, maximal jedoch auf 180°C erwärmt wird, und
daß das optische Grundelement maximal 30 min in die Mischung getaucht wird.

## Claims

1. Compound corresponding to the general formula 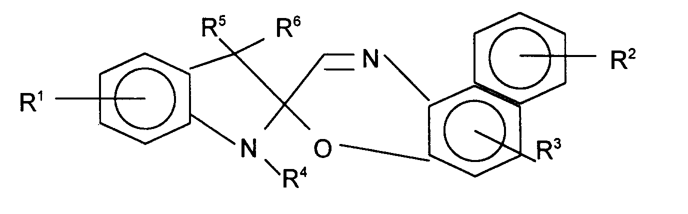 wherein
R¹, R², R³ represent one or several substituents from the series -H, -CN, -NO₂, -X, -CH₂X, -CH₂X, -CX₃ -OR, -COR, -COOR,
with X = a halogen and R = alkyl with up to 8 carbon atoms.
R⁴, R⁵, R⁶ are each a substituent from the series -R, -CN₂R,
with R = an alkyl having up to 8 carbon atoms or an aryl whilst the radicals R⁶ and R⁶ are not both methyl.

2. Compound corresponding to the following formula:

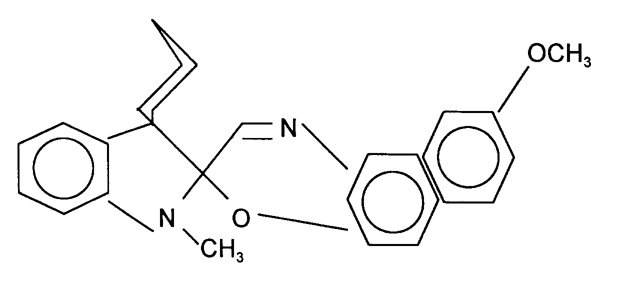

3. Optical element,
**characterised** in that it contains an efficient quantity of a photochromic compound according to Claim 1 or 2 which is introduced into the optical element or is applied as a layer on a plastic carrier material.

4. Optical element according to Claim 3,
**characterised** in that the optical element consists of a synthetic material.

5. Optical element according to Claim 3 or 4,
**characterised** in that the element is manufactured in a way
that said photochromic compound is stirred into a silicone oil or a similar inert high-boiling solvent,
that the mixture is heated to a temperature roughly 10° above the melting point of said photochromic compound, however to a temperature not exceeding 180 °C at maximum, and
that the optical basic element is immersed into the mixture for 30 minutes at maximum.

## Revendications

1. Composé correspondant à la formule de structure générale: 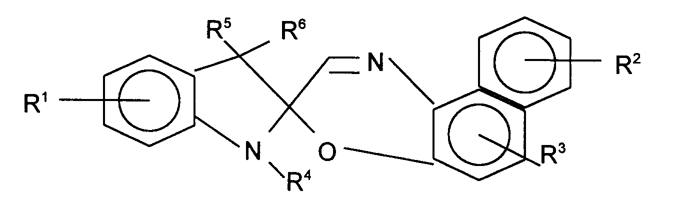 dans laquelle
R¹, R², R³ représente un ou plusieurs substituants de la série -H, -CN, -NO₂, -X, -CH₂X, -CH₂X, -CX₃, -OR, -COR, -COOR,
à X = un halogène et R = un alkyle à jusqu'à 8 atomes de carbone;
R⁴, R⁵, R⁶ sont chacun un substituant de la série -R, -CH₂R,
à R = un alkyle à jusqu'à 8 atomes de carbone ou un aryle, pendant que les radicaux R⁶ et R⁶ ne sont pas de méthyle tous les deux.

2. Composé correspondant à la formule de structure générale:

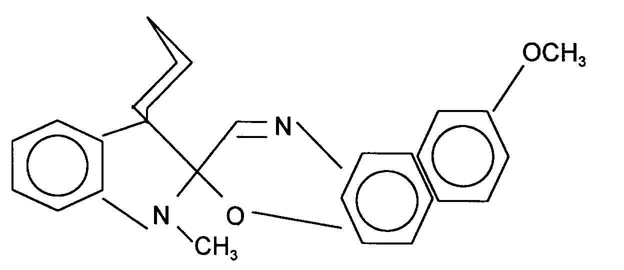

3. Elément optique,
**caractérisé** en ce qu'il contient une quantité active d'un composé photosensible selon la revendication 1 ou 2, qui est incorporée dans l'élément optique ou qui est appliquée comme une couche sur un matériau de support synthétique.

4. Elément optique selon la revendication 3
**caractérisé** en ce que l'élément optique consiste en un matériau synthétique.

5. Elément optique selon la revendication 3 ou 4,
**caractérisé** en ce que l'élément est produit de façon
que ledit composé photosensible est agité dans une huile de silicone ou un solvant inerte similaire à point d'ébullition élevé,
que le mélange est chauffé à une température plus haute, par 10° environ, que le point de fusion dudit composé photosensible, mais à un niveau que ne dépasse pas le maximum de 180 °C, et
que l'élément optique de base est plongé dans le mélange pour un intervalle de 30 minutes au maximum.
